# EUROPEAN PATENT APPLICATION

(11) **EP 3 424 485 A1**
(43) Date of publication of application: **09.01.2019**
(21) Application number: 17760098.8
(22) Date of filing: 01.03.2017
(51) Int. Cl.: A61K 6/08, A61C 8/00, A61C 13/087, A61C 13/10, B82Y 30/00

(54) **DENTAL MATERIAL**

(30) Priority: 01.03.2016 JP 2016039051
(71) Applicant: Kuriyama, Takuya, Nishinomiya-shi, Hyogo 663-8211 (JP); Celumix Inc., Ashiya-shi, Hyogo 6590016 (JP)
(72) Inventor: KURIYAMA, Takuya, Hyogo 6638211 (JP)
(74) Representative: Sackin, Robert
(86) International application number: PCT/JP2017/008181
(87) International publication number: WO 2017/150634

(57) **Abstract**

The present invention provides a dental material containing nanofibers and a biocompatible resin. In one embodiment, this dental material is an implant material, prosthetic material, or denture material and has mechanical strength suited to the site of use in the body as well as exceptional dimensional stability and exceptional wear resistance. Because the compressive strength can be controlled, excessive burden on the teeth when occlusal force is applied is suppressed when the dental material is used in the oral cavity. In addition, secondary caries can be suppressed because the antimicrobial properties make plaque less likely to adhere to the teeth and make the teeth less likely to be affected by bacteria. This dental material can be used without modification as a desired dental material, and can also be made into a desired dental material by molding as needed. In addition, materials produced from these nanofibers and this resin can also be used in applications other than dental materials.

## Description

### [Technical Field]

The present invention relates to a dental material used in dental care.

### [Background Art]

The oral environment is a special environment, where the pH changes from intake of acidic or alkaline food, the temperature changes from intake of hot or cold food, breathing induces a partially dried state despite being moist due to saliva, a significant occlusal force and impact involving mastication are applied, occlusal wear due to occlusion or mastication and wear due to brushing or the like occur, plaque buildup enables the environment to be a hotbed of microbes, saliva acts as an electrolyte to facilitate electrochemical changes, and the like. Due to such special properties, dental materials used inside the oral cavity are required to have a high level of biocompatibility, biologically suitable mechanical strength (resistance to occlusal force in the oral cavity), and durability.

For example, metal materials such as titanium are used as dental implant materials in view of their durability, biocompatibility, or high compressive strength. However, metal materials such as titanium generally have a mechanical strength that is too high, which can have an adverse effect on the human body such as a fracture in the crown or root of the opposing tooth.

In recent years, development of novel materials focusing on nanofibers as an organic resource derived from organisms has been attempted. In particular, cellulose, which is the main component of a plant cell wall, is considered in applications in various fields due to its abundance as a resource, excellent durability and strength, and the like.

For example, a resin composition with high mechanical strength and excellent impact strength as well as moldability that is prepared by including thermoplastic resin, cellulose fiber, and inorganic matter at a predetermined ratio has been proposed for use in obtaining a molding that is useful especially as an automotive component (see Patent Literature 1). Further, a dry film with high strength (elastic modulus or rupture strength) that can be formed with a resin composition comprising a cellulose fiber and resin emulsion satisfying a specified condition has been proposed to be applied as an adhesive, paint, wax, or a raw material for the manufacture thereof by utilizing the film forming action (see Patent Literature 2).

### [Citation List]

### [Patent Literature]

[PTL 1] Japanese Laid-Open Publication No. 2012-201767
[PTL 2] Japanese Laid-Open Publication No. 2009-197122

### [Summary of Invention]

### [Technical Problem]

The present invention has been conceived in view of the above circumstances. The objective of the present invention is to provide a dental material with biocompatibility, biologically suitable mechanical strength such as resistance to occlusal force in the oral cavity, and durability.

### [Solution to Problem]

The inventors have unexpectedly found that a dental material with biocompatibility, biologically suitable mechanical strength such as resistance to occlusal force in the oral cavity, and durability is provided by applying nanofibers (especially cellulose nanofibers), which have not been applied in the field of dental materials, to dental materials.

The resin compositions, as such those described in Patent Literatures 1 and 2, use nanofibers as a resin reinforcement material while primarily envisioning industrial applications. Thus, such materials were not applicable to dental materials to begin with. Specifically, materials with a strength required in industrial applications are too hard as a dental material and lack properties that match a special environment inside the oral cavity. For this reason, there has been a problem, when applied as a dental material, of a potential defect due the dental material itself fracturing or coming off due to the action of occlusal force from occlusion or mastication or the like. There have been problems not only in the dental material itself, but also in the tooth or tissue to which the dental material is applied, tissue adjacent to the dental material and the like, which can potentially have a fracture or symptom such as inflammation due to compatibility with the dental material, difference in physical properties, or the like.

While various thermoplastic resins and thermosetting resins are acceptable for use in industrial applications, it was impossible to use phenol resins and the like that are commonly and frequently used as a raw material of a dental material used inside the oral cavity due to the potential carcinogenicity.

Thus, it was unexpected that nanofibers (especially cellulose nanofibers) can be applied to dental materials, whereby a dental material with biocompatibility, biologically suitable mechanical strength such as resistance to occlusal force in the oral cavity, and durability can be provided.

The dental material of the present invention is characterized by being a dental material comprising a nanofiber and a biocompatible resin.

In one embodiment, the nanofiber is a cellulose nanofiber or a cellulose nanofiber composite.

In one embodiment, the nanofiber further comprises one or more of a chitosan nanofiber and a chitin nanofiber.

In one embodiment, the dental material comprises an antimicrobial substance.

In one embodiment, the dental material is an implant material, a prosthetic material, a denture material, a filler, a denture plate material, a mold repairing material, or an impression material.

In one embodiment, the dental material is an implant material, a prosthetic material, or a denture material with a compressive strength within a range of 300 MPa to 400 MPa.

In one embodiment, the dental material is a filler with a compressive strength within a range of 150 MPa to 250 MPa.

In one embodiment, the dental material is a denture plate material with a compressive strength within a range of 60 MPa to 100 MPa.

In one embodiment, the dental material is a mold repairing material or an impression material with a compressive strength within a range of 30 MPa to 50 MPa.

For example, the present invention provides the following.

### (Item 1)

A dental material comprising a nanofiber and a biocompatible resin.

### (Item 2)

The dental material of item 1, wherein the nanofiber is a cellulose nanofiber.

### (Item 3)

The dental material of item 2, wherein the nanofiber further comprises one or more of a chitosan nanofiber and a chitin nanofiber.

### (Item 4)

The dental material of any one of items 1 to 3, wherein the dental material comprises an antimicrobial substance.

### (Item 5)

The dental material of any one of items 1 to 4, wherein the dental material is an implant material, a prosthetic material, a denture material, a filler, a denture plate material, a mold repairing material, or an impression material.

### (Item 6)

The dental material of item 5, wherein the dental material is an implant material, a prosthetic material, or a denture material with a compressive strength within a range of 300 MPa to 400 MPa.

### (Item 7)

The dental material of item 5, wherein the dental material is a filler with a compressive strength within a range of 150 MPa to 250 MPa.

### (Item 8)

The dental material of item 5, wherein the dental material is a denture plate material with a compressive strength within a range of 60 MPa to 100 MPa.

### (Item 9)

The dental material of item 5, wherein the dental material is a mold repairing material or an impression material with a compressive strength within a range of 30 MPa to 50 MPa.

### (Item 10)

The dental material of any one of items 6 to 9, wherein the compressive strength is obtained by testing in accordance with one or more specifications selected from JIS T6123, JIS T6501, JIS T6502, JIS T6503, JIS T6505, JIS T6506, JIS T6508, JIS T6509, JIS T6512, JIS T6513, JIS T6514, JIS T6515, JIS T6517, JIS T6518, JIS T6519, JIS T6520, JIS T6521, JIS T6522, JIS T6523, JIS T6524, JIS T6525-1, JIS T6525-2, JIS T6527, JIS T6601, JIS T6604, JIS T6605, JIS T6608, JIS T6609-1, JIS T6609-2, JIS T6610, JIS T6611, JIS T6612, JIS T6003, JIS T6005, and JIS K 7181.

### (Item 11)

A material comprising a nanofiber and a resin, further comprising a substance selected from the group consisting of :
(1) a coating agent and
(2) an additive selected from the group consisting of lead, tungsten, boron, graphite, graphene, and cadmium.

### (Item 12)

The material of item 11 for strengthening a cask of a fuel rod, or for use as a radiation shield, as a reinforcing material for a body of an automobile, a ship, a spacecraft, a space station, a rocket, an aircraft, or a motorcycle, as a space debris post-pulverization adhesive material, as a material for a debris bumper equipped space craft or space station, as a material for a robot frame, as a roofing material comprising a radiation shielding material, or as a construction material.

### (Item 13)

The material of item 11, wherein the nanofiber is a cellulose nanofiber.

### (Item 14)

The material of item 11, wherein the resin is selected from the group consisting of methyl methacrylate (MMA), polymethyl methacrylate (PMMA), 2-hydroxyethyl methacrylate (HEMA), tri-n-butylborane (TBB), and 4-methacryloxyethyl trimellitic anhydride (4-META).

### (Item 15)

The material of item 11, wherein the coating agent is polyuria.

### (Item 16)

The material of any one of items 11 to 15 with a compressive strength within a range of 1 GPa to 3 GPa.

### (Item 17)

The dental material of item 16, wherein the compressive strength is obtained by testing in accordance with one or more specifications selected from JIS A1106:2006, JIS A1107:2012, JIS A1108:2006, JIS A1113:2006, JIS A1114:2011, JIS A1132:2014, JIS A1136:1993, JIS A1142:2007, JIS D4610:1993, JIS H7701:2008, JIS R3222:2003, JIS S1200:2012, JIS S1203:1998, JIS S1205:1998, JIS Z8841:1:1993, AST MD953-95, ISO/TS 20746:2016, ISO 75-3:2004, ISO 1752020:2016, ISO 1920-4:2005, ISO 1920-5:2004, ISO 2633:1974, ISO 3185:2008, ISO 3186:2008, ISO 3193:2008, ISO-3202:1997, ISO-3203:1993, ISO-7689:2008, ISO-8168:2016, ISO-5856:2008, ISO-5857:2008, ISO-7173:1989, ISO-7176:8:2014, ISO-9152:1998, ISO-9154:2016, ISO-9254:1993, ISO-9255:2008, ISO-9256:1993, ISO-9709:2005, ISO-9845-1:1992, ISO-12258:1998, ISO-12260:2016, ISO-12261:2016, and ISO-1391:2008.

### [Advantageous Effects of Invention]

The present invention provides a dental material with excellent resistance to occlusal force in the oral cavity, biologically suitable mechanical strength, and durability.

### [Description of Embodiments]

The embodiments of the present invention are explained hereinafter in more detail.

### <Definitions>

As used herein, "dental material" encompasses materials used inside the oral cavity and materials for making the same. Dental materials applied in the oral cavity (e.g., implant material, prosthetic material, denture material, filler, denture plate material, or mold repairing material) refer to materials, which is directly or indirectly subjected to an occlusal force from occlusion or mastication at least on a portion thereof, when applied within the oral cavity.

More specific examples of the dental materials of the present invention include, but are not limited to, implant materials, prosthetic materials (inlay, crown, core, bridge, and the like), denture materials (artificial teeth), denture plate materials (including denture plate stabilizers, denture plate liners and the like), mold repairing materials (root canal fillers, gutta-percha, pit and fissure sealants, other sealers, sealants, and the like), impression materials (mold making materials), fillers (filler resins and the like), modeling materials, wax, dental investment materials, cementing materials, and the like.

As used herein, "nanofiber" refers to a fibrous substance with a nanoscale mean outer diameter value within the range of 1 nm to 100 nm and a length that is 100-fold or greater of the mean outer diameter value. Examples of nanofibers include nanotubes with a hollow structure, nanorods without a hollow structure, nanowires with an electrically conductive or semi-conductive property, and the like.

As used herein, "biocompatible" means that an adverse effect is not exerted at least beyond the desired intended effect in terms of the interaction between a dental material and biological tissue, and topical and systemic reactions of tissue adjacent to a dental material.

More specifically, biocompatibility of an implant materia, when the dental material is an implant material, refers to for example a property allowing biological tissue to recover a function and/or heal at the interface between the implant material and biological tissue to which the implant material is applied, so that the implant material can provide a state where the functionality is maintained. Biocompatibility of a dental material can be evaluated, for example, from the viewpoint of surface (interface) compatibility or the viewpoint of mechanical compatibility.

As used herein, "implant material" refers to a material that is embedded and used under the mucous membrane in the oral cavity or inside the jaw bone. Examples of implant materials include, but are not limited to, intraosseous implants, subperiosteal implants, endodontic intraosseous implants, and the like.

As used herein, "prosthetic material" refers to a material that is used to repair a defective portion of a tooth. Examples of prosthetic materials include, but are not limited to, inlays, crowns, cores, bridges, and the like.

### <A. Dental material>

The material comprising a nanofiber and a biocompatible material of the present invention is excellent as a dental material. In one embodiment, the dental material of the present invention comprises a nanofiber and a biocompatible resin.

### (1. Nanofiber)

A white or milky white nanofiber is preferably used especially from the viewpoint of aesthetics. Since a cellulose nanofiber, for example, is a renewable natural resource with biocompatibility, a cellulose nanofiber can be preferably used from the viewpoint of environmental protection or recycling of resources in addition to the viewpoint of attaining the required characteristics as a dental material.

Cellulose nanofibers are not particularly limited. Commercially available products or products manufactured by a known manufacturing method may be used. Examples of raw materials that are used in manufacturing a cellulose nanofiber include plant derived fibers contained in timber, bamboo, hemp, jute, kenaf, cotton, beet, or the like. Preferred examples of raw materials include timber such as pine, Japanese cedar, Japanese cypress, Eucalyptus, and acacia. Paper or used paper obtained with such timber as the raw material or the like can also be used. One type of plant derived fiber may be used alone or two or more types may be used concurrently.

Examples of cellulose nanofibers include pulp obtained from the plant fiber containing materials, mercerized cellulose nanofibers, rayon and cellophane, recycled cellulose nanofibers such as lyocell, and the like. Use of cellulose nanofibers obtained from scrap material or agricultural waste is considered preferable from the viewpoint of recycling of resources.

As a cellulose nanofiber, a composite of a cellulose nanofiber and another substance (referred to as "cellulose nanofiber composite" herein) can also be used depending on the type, application, required property or the like of a dental material. Examples of other materials that are used to form a composite with a cellulose nanofiber include calcium carbonate, tricalcium phosphate, calcium sulfate, and the like. Use of a composite of a cellulose nanofiber and calcium carbonate and a composite of a cellulose nanofiber and tricalcium phosphate is particularly considered preferable. One type of cellulose nanofiber may be used alone or two or more types may be used concurrently.

For the dental material according to this embodiment, one type of nanofiber may be used alone or two or more types may be used concurrently. In one embodiment, it is preferable that a nanofiber further comprises one or more of a chitosan nanofiber and a chitin nanofiber. The antimicrobial property of a dental material can be further improved thereby. In this manner, the type or combination of nanofibers can be appropriately selected depending on the type, application, required property or the like of a dental material.

### (2. Biocompatible resin)

Examples of biocompatible resins include, but are not limited to, monomers, oligomers and polymers of acrylic acid, acrylic acid ester, methacrylic acid, methacrylic acid ester, carbonate, propylene, styrene, amide, imide, glycolic acid, lactic acid, maltose, and dextrin, and the like. More specific examples of biocompatible resins include, but are not limited to, methyl methacrylate (MMA), polymethyl methacrylate (PMMA); alkyl esters of methacrylic acid such as ethyl methacrylate (EMA), n-butyl methacrylate, isobutyl methacrylate, tert-butyl methacrylate, 2-ethylhexyl methacrylate, n-lauryl methacrylate, alkyl (C12-13) methacrylate, n-stearyl methacrylate, and tridecyl methacrylate; dialkylaminoethyl esters of methacrylic acid such as dimethylaminoethyl methacrylate, methyl chloride salt of dimethylaminoethyl methacrylate, benzyl chloride salt of dimethylaminoethyl methacrylate, and diethylaminoethyl methacrylate; carboxylic acid-containing esters of methacrylic acid such as methacryloxyethyl phthalate, 2-methacryloyloxyethyl phthalate, and 2-methacryloyloxyethyl hexahydrophthalate; fluoroalkyl esters of methacrylic acid such as 2,2,2-trifluoroethyl methacrylate; cyclohexyl methacrylate, phenyl methacrylate, benzyl methacrylate, isobornyl methacrylate, glycidyl methacrylate, tetrahydrofurfuryl methacrylate, allyl methacrylate, 2-hydroxyethyl methacrylate (HEMA), 2-hydroxypropyl methacrylate, 2-methoxyethyl methacrylate, 2-ethoxyethyl methacrylate, hydroxy naphthoxypropyl methacrylate (HNPM), ethylene glycol dimethacrylate (EDMA), triethylene glycol methacrylate (TriEDMA), 1,3-butanediol dimethacrylate (1,3-BuDMA), 1,3-butylene glycol dimethacrylate, 1,6-hexanediol dimethacrylate, polypropylene glycol methacrylate, trimethylolpropane trimethacrylate, 2,2-bis[4-(2-hydroxy-3-methacryloxypropoxy)phenyl]propane (Bis-GMA), 2,2-bis(4-methacryloxyphenyl)propane (BPDMA), 2,2-bis(4-methacryloxyethoxyphenyl)propane (Bis-MEPP), di(methacryloxyethyl)trimethylhexamethylene diurethane (UDMA), tri-n-butylborane (TBB), methacryloxyethyl phenyl phosphate (Phenyl-P), 4-methacryloxyethyl trimellitic anhydride (4-META), 4-methacryloxyethyl trimellitic acid (4-MET), 11-methacryloxy-1,1-undecadicarboxylic acid (MAC-10), 10-methacryloxydecamethylene phosphate (MDP), 4-acryloxyethyl trimellitic acid (4-AET), and the like. It is considered preferable to use especially MMA, PMMA, HEMA, TBB, or 4-META. One type of biocompatible resin may be used alone or two or more types may be used concurrently.

### (3. Antimicrobial substance)

The dental material according to this embodiment also preferably comprises an antimicrobial substance. The antimicrobial property of a dental material can be further improved thereby. The antimicrobial substance is not particularly limited, as long as it is a substance with antimicrobial activity that does not have an adverse effect on the human body. An antimicrobial substance can be appropriately selected depending on the type, application, required property or the like of a dental material. Examples thereof include, but are not limited to, chitosan (including chitosan derivatives), chitin (including chitin derivatives), agents with antimicrobial activity, metal ions (e.g., copper ions, silver ions, and the like), and the like. When an agent with antimicrobial activity, metal ion or the like is used as an antimicrobial substance, it is considered preferable to encapsulate such an antimicrobial substance in a microcapsule for inclusion in a dental material from the viewpoint of exerting the antimicrobial property of the dental material more effectively.

### (4. Other additives)

A glidant, wax, colorant, stabilizer, filler, and other various additives can be optionally included in the dental material according to this embodiment, to the extent that the effect of the present invention is not inhibited. For example, a compatibilizer may be added to a dental material to facilitate the mixture of a nanofiber and biocompatible resin. A colorant may be optionally added to a dental material to match the color tone of biological tissue to which the dental material is applied and/or tissue adjacent to the dental material or the like.

### (5. Modification)

Modification such as surface modification can be optionally applied to the dental material according to this embodiment depending on the type, application, required property or the like of the dental material. For example, hydrophilicity/hydrophobicity of a dental material can be adjusted by coating a part or the entire surface of the dental material with a hydrophilic or hydrophobic substance.

### (6. Properties)

The mechanical property, physical property, chemical property, biological stability, mechanical stability and the like of the dental material of the present invention can be evaluated using the following representative items as an indicator.

### (6-1. Compressive strength, tensile strength, and extension)

The compressive strength (MPa), tensile strength (MPa), and/or extension (%) are measured by applying a linear load on the target dental material in accordance with a common compression testing method and tensile testing method. The compressive strength in the present invention is a value obtained by testing a sample of a target dental material in accordance with one or more specifications selected from JIS T6123, JIS T6501, JIS T6502, JIS T6503, JIS T6505, JIS T6506, JIS T6508, JIS T6509, JIS T6512, JIS T6513, JIS T6514, JIS T6515, JIS T6517, JIS T6518, JIS T6519, JIS T6520, JIS T6521, JIS T6522, JIS T6523, JIS T6524, JIS T6525-1, JIS T6525-2, JIS T6527, JIS T6601, JIS T6604, JIS T6605, JIS T6608, JIS T6609-1, JIS T6609-2, JIS T6610, JIS T6611, JIS T6612, JIS T6003, JIS T6005, and JIS K 7181. For compressive strength, the same testing method may be applied regardless of the type of dental material, or different testing methods may be applied depending on the type of dental material.

Since the dental material of the present invention is a dental material comprising a nanofiber and a biocompatible resin, the dental material has excellent resistance to occlusal force in the oral cavity. It is also possible to suitably control the compressive strength of the dental material of the present invention, so that fracture of a tooth root due to a compressive strength that is too high can be prevented. The dental material of the present invention is also better compared to conventional dental materials with respect to this point.

### (6-2. Elastic modulus and yield value)

The elastic modulus (GPa) and/or yield value (MPa) of a target dental material is measured with any testing apparatus in accordance with a common testing method.

Since the dental material of the present invention is a dental material comprising a nanofiber and a biocompatible resin, the dental material can be manufactured to have the same or better elastic modulus and/or yield value compared to conventional dental materials.

### (6-3. Flexural strength and flexural modulus)

The flexural strength (MPa) and/or flexural modulus (MPa) of a target dental material is measured in accordance with JIS T6501 or the like.

Since the dental material of the present invention is a dental material comprising a nanofiber and a biocompatible resin, the dental material can be manufactured to have the same or better flexural strength and/or flexural modulus compared to conventional dental materials.

### (6-4. Impact strength)

The impact strength (kgf·cm/cm) of a target dental material is measured by a commonly known Charpy method, Izod method, or the like.

Since the dental material of the present invention is a dental material comprising a nanofiber and a biocompatible resin, the dental material can be manufactured to have the same or better impact strength compared to conventional dental materials.

### (6-5. Hardness)

The Brinell hardness (H_{B}), Vickers hardness (H_{V}), Knoop hardness (H_{K}), Shore hardness, or the like of a target dental material is measured by a commonly known indentation method, elasticity method, scratching method or the like.

Since the dental material of the present invention is a dental material comprising a nanofiber and a biocompatible resin, the dental material can be manufactured to have the same or better hardness compared to conventional dental materials.

### (6-6. Fracture toughness)

The fracture toughness (MN/m^{3/2}) of a target dental material is measured by a commonly known IM method, CSF, method, CN method, SENB method or the like.

Since the dental material of the present invention is a dental material comprising a nanofiber and a biocompatible resin, the dental material can be manufactured to have the same or better fracture toughness compared to conventional dental materials.

### (6-7. Dimensional stability)

The dimension test of a target dental material is performed in accordance with JIS T6506 or the like.

Since the dental material of the present invention is a dental material comprising a nanofiber and a biocompatible resin, the dental material has better dimensional stability compared to conventional dental materials. The shape, size, and the like after hardening can also be more accurately controlled by making an implant material, denture material or the like with a dental material of the same component as the impression material (mold making material) in accordance with the present invention.

### (6-8. Durability)

The antiwear property of a target dental material is measured by a commonly known wear/friction testing instrument or the like.

Since the dental material of the present invention is a dental material comprising a nanofiber and a biocompatible resin, the dental material has a better antiwear property compared to conventional dental materials.

### (6-9. Thermal expansion)

The linear coefficient of expansion (× 10⁻⁶/°C) or the like of a target dental material is measured with a thermal expansion testing instrument or the like in accordance with a common thermal expansion testing method.

Since the dental material of the present invention is a dental material comprising a nanofiber and a biocompatible resin, a change in dimension is suppressed.

### (6-10. Solubility)

The solubility of a target dental material in acid/alkali is measured in accordance with a commonly known acid/alkali solubility test or the like.

Since the dental material of the present invention is a dental material comprising a nanofiber and a biocompatible resin, the dental material has excellent chemical stability against both acid and alkali.

### (6-11. Water absorption rate)

The water absorption rate (%) of a target dental material is measured in accordance with JIS T6501 or the like.

Since the dental material of the present invention is a dental material comprising a nanofiber and a biocompatible resin wherein the nanofiber and the biocompatible resin form a tight composite, the dental material has the same or better water resistance compared to conventional dental materials.

### (6-12. Porosity and pore diameter)

The porosity (%) and/or pore diameter (µm or nm) is measured on any cross-section of a target dental material by electron microscope observation.

Since the dental material of the present invention is a dental material comprising a nanofiber and a biocompatible resin wherein the nanofiber and the biocompatible resin form a tight composite, the dental material has very low porosity.

### (6-13. Outward appearance evaluation)

The outward appearance of a target dental material is observed by visual inspection, using a magnifying glass, or the like.

Since the dental material of the present invention is a dental material comprising a nanofiber and a biocompatible resin wherein the nanofiber and the biocompatible resin form a tight composite, the dental material has good aesthetics due to having a similar type of color as the tooth and excellent outward appearance.

### (6-14. Color tone stability)

The color tone stability of a target dental material is measured in accordance with JIS T6003.

Since the dental material of the present invention is a dental material comprising a nanofiber and a biocompatible resin wherein the nanofiber and the biocompatible resin form a tight composite, the dental material has better color tone stability and retains the aesthetics for a longer period of time compared to conventional dental materials. Since the dental material of the present invention is a white material with high transparency, a desired color tone can be readily achieved by adjusting the type and amount of colorant that is added during the manufacture.

### (6-15. Biocompatibility)

The biocompatibility of a target dental material is tested in accordance with "Biological evaluation of medical devices" testing in ISO 10993, USP/USP class VI biological testing, or the like.

Since the dental material of the present invention is a dental material comprising a nanofiber and a biocompatible resin, the dental material has the same or better biocompatibility compared to conventional dental materials.

### (7. Specific examples of dental materials)

Implant materials, prosthetic materials, denture materials, fillers, denture plate materials, mold repairing materials, and impression materials are explained below as specific embodiments of the dental material of the present invention.

### (7-1. Implant material, prosthetic material, denture material)

In one embodiment, the dental material of the present invention is an implant material, a prosthetic material, or a denture material with a compressive strength within a range of 300 MPa to 400 MPa. In one aspect of this embodiment, a compressive strength within a range of 300 MPa to 400 MPa is obtained by testing in accordance with JIS T6123. In another aspect of this embodiment, a compressive strength within a range of 300 MPa to 400 MPa is obtained by testing in accordance with JIS T6501. In another aspect of this embodiment, a compressive strength within a range of 300 MPa to 400 MPa is obtained by testing in accordance with JIS T6502. In another aspect of this embodiment, a compressive strength within a range of 300 MPa to 400 MPa is obtained by testing in accordance with JIS T6503. In another aspect of this embodiment, a compressive strength within a range of 300 MPa to 400 MPa is obtained by testing in accordance with JIS T6505. In another aspect of this embodiment, a compressive strength within a range of 300 MPa to 400 MPa is obtained by testing in accordance with JIS T6506. In another aspect of this embodiment, a compressive strength within a range of 300 MPa to 400 MPa is obtained by testing in accordance with JIS T6508. In another aspect of this embodiment, a compressive strength within a range of 300 MPa to 400 MPa is obtained by testing in accordance with JIS T6509. In another aspect of this embodiment, a compressive strength within a range of 300 MPa to 400 MPa is obtained by testing in accordance with JIS T6512. In another aspect of this embodiment, a compressive strength within a range of 300 MPa to 400 MPa is obtained by testing in accordance with JIS T6513. In another aspect of this embodiment, a compressive strength within a range of 300 MPa to 400 MPa is obtained by testing in accordance with JIS T6514. In another aspect of this embodiment, a compressive strength within a range of 300 MPa to 400 MPa is obtained by testing in accordance with JIS T6515. In another aspect of this embodiment, a compressive strength within a range of 300 MPa to 400 MPa is obtained by testing in accordance with JIS T6517. In another aspect of this embodiment, a compressive strength within a range of 300 MPa to 400 MPa is obtained by testing in accordance with JIS T6518. In another aspect of this embodiment, a compressive strength within a range of 300 MPa to 400 MPa is obtained by testing in accordance with JIS T6519. In another aspect of this embodiment, a compressive strength within a range of 300 MPa to 400 MPa is obtained by testing in accordance with JIS T6520. In another aspect of this embodiment, a compressive strength within a range of 300 MPa to 400 MPa is obtained by testing in accordance with JIS T6521. In another aspect of this embodiment, a compressive strength within a range of 300 MPa to 400 MPa is obtained by testing in accordance with JIS T6522. In another aspect of this embodiment, a compressive strength within a range of 300 MPa to 400 MPa is obtained by testing in accordance with JIS T6523. In another aspect of this embodiment, a compressive strength within a range of 300 MPa to 400 MPa is obtained by testing in accordance with JIS T6524. In another aspect of this embodiment, a compressive strength within a range of 300 MPa to 400 MPa is obtained by testing in accordance with JIS T6525-1. In another aspect of this embodiment, a compressive strength within a range of 300 MPa to 400 MPa is obtained by testing in accordance with JIS T6525-2. In another aspect of this embodiment, a compressive strength within a range of 300 MPa to 400 MPa is obtained by testing in accordance with JIS T6527. In another aspect of this embodiment, a compressive strength within a range of 300 MPa to 400 MPa is obtained by testing in accordance with JIS T6601. In another aspect of this embodiment, a compressive strength within a range of 300 MPa to 400 MPa is obtained by testing in accordance with JIS T6604. In another aspect of this embodiment, a compressive strength within a range of 300 MPa to 400 MPa is obtained by testing in accordance with JIS T6605. In another aspect of this embodiment, a compressive strength within a range of 300 MPa to 400 MPa is obtained by testing in accordance with JIS T6608. In another aspect of this embodiment, a compressive strength within a range of 300 MPa to 400 MPa is obtained by testing in accordance with JIS T6609-1. In another aspect of this embodiment, a compressive strength within a range of 300 MPa to 400 MPa is obtained by testing in accordance with JIS T6609-2. In another aspect of this embodiment, a compressive strength within a range of 300 MPa to 400 MPa is obtained by testing in accordance with JIS T6610. In another aspect of this embodiment, a compressive strength within a range of 300 MPa to 400 MPa is obtained by testing in accordance with JIS T6611. In another aspect of this embodiment, a compressive strength within a range of 300 MPa to 400 MPa is obtained by testing in accordance with JIS T6612.

In another aspect of this embodiment, a compressive strength within a range of 300 MPa to 400 MPa is obtained by testing in accordance with JIS T6003. In another aspect of this embodiment, a compressive strength within a range of 300 MPa to 400 MPa is obtained by testing in accordance with JIS T6005. In another aspect of this embodiment, a compressive strength within a range of 300 MPa to 400 MPa is obtained by testing in accordance with JIS K 7181. More specifically, a compressive strength within a range of 300 MPa to 400 MPa in the denture material according to this embodiment is obtained, for example, by testing in accordance with JIS T6517. A compressive strength within a range of 300 MPa to 400 MPa in the denture material according to this embodiment is also obtained by testing in accordance with JIS T6518. A compressive strength within a range of 300 MPa to 400 MPa in the denture material according to this embodiment is also obtained by testing in accordance with JIS T6525-1. A compressive strength within a range of 300 MPa to 400 MPa in the denture material according to this embodiment is also obtained by testing in accordance with JIS T6525-2.

In this embodiment, the nanofiber content with respect to the total mass of the dental material is preferably within the range of 70% to 90%, more preferably within the range of 75% to 90%, and still more preferably within the range of 80% to 85%. Further, the ratio of biocompatible resin among components other than the nanofiber in the dental material is preferably at least 90% or greater. When an antimicrobial substance and other additives are added, the content thereof can be appropriately determined depending on the type, application, required property or the like of a dental material.

The dental material can thereby be an implant material, prosthetic material, or denture material, which has a mechanical strength that is suitable to the site within the organism where the implant material, prosthetic material, or denture material, has excellent dimensional stability, and has excellent antiwear property. Since the compressive strength of the implant material, prosthetic material, or denture material according to this embodiment is controllable, excessive load on a tooth when occlusal force is applied is suppressed when such a material is applied to the oral cavity. Since the antimicrobial property of the implant material, prosthetic material, or denture material according to this embodiment makes it unlikely that a tooth has plaque adhering thereto or is affected by microbes, secondary tooth decay can also be suppressed.

### (7-2. Filler)

In one embodiment, the dental material of the present invention is a filler with a compressive strength within a range of 150 MPa to 250 MPa. In one aspect of this embodiment, a compressive strength within a range of 150 MPa to 250 MPa is obtained by testing in accordance with JIS T6123. In another aspect of this embodiment, a compressive strength within a range of 150 MPa to 250 MPa is obtained by testing in accordance with JIS T6501. In another aspect of this embodiment, a compressive strength within a range of 150 MPa to 250 MPa is obtained by testing in accordance with JIS T6502. In another aspect of this embodiment, a compressive strength within a range of 150 MPa to 250 MPa is obtained by testing in accordance with JIS T6503. In another aspect of this embodiment, a compressive strength within a range of 150 MPa to 250 MPa is obtained by testing in accordance with JIS T6505. In another aspect of this embodiment, a compressive strength within a range of 150 MPa to 250 MPa is obtained by testing in accordance with JIS T6506. In another aspect of this embodiment, a compressive strength within a range of 150 MPa to 250 MPa is obtained by testing in accordance with JIS T6508. In another aspect of this embodiment, a compressive strength within a range of 150 MPa to 250 MPa is obtained by testing in accordance with JIS T6509. In another aspect of this embodiment, a compressive strength within a range of 150 MPa to 250 MPa is obtained by testing in accordance with JIS T6512. In another aspect of this embodiment, a compressive strength within a range of 150 MPa to 250 MPa is obtained by testing in accordance with JIS T6513. In another aspect of this embodiment, a compressive strength within a range of 150 MPa to 250 MPa is obtained by testing in accordance with JIS T6514. In another aspect of this embodiment, a compressive strength within a range of 150 MPa to 250 MPa is obtained by testing in accordance with JIS T6515. In another aspect of this embodiment, a compressive strength within a range of 150 MPa to 250 MPa is obtained by testing in accordance with JIS T6517. In another aspect of this embodiment, a compressive strength within a range of 150 MPa to 250 MPa is obtained by testing in accordance with JIS T6518. In another aspect of this embodiment, a compressive strength within a range of 150 MPa to 250 MPa is obtained by testing in accordance with JIS T6519. In another aspect of this embodiment, a compressive strength within a range of 150 MPa to 250 MPa is obtained by testing in accordance with JIS T6520. In another aspect of this embodiment, a compressive strength within a range of 150 MPa to 250 MPa is obtained by testing in accordance with JIS T6521. In another aspect of this embodiment, a compressive strength within a range of 150 MPa to 250 MPa is obtained by testing in accordance with JIS T6522. In another aspect of this embodiment, a compressive strength within a range of 150 MPa to 250 MPa is obtained by testing in accordance with JIS T6523. In another aspect of this embodiment, a compressive strength within a range of 150 MPa to 250 MPa is obtained by testing in accordance with JIS T6524. In another aspect of this embodiment, a compressive strength within a range of 150 MPa to 250 MPa is obtained by testing in accordance with JIS T6525-1. In another aspect of this embodiment, a compressive strength within a range of 150 MPa to 250 MPa is obtained by testing in accordance with JIS T6525-2. In another aspect of this embodiment, a compressive strength within a range of 150 MPa to 250 MPa is obtained by testing in accordance with JIS T6527. In another aspect of this embodiment, a compressive strength within a range of 150 MPa to 250 MPa is obtained by testing in accordance with JIS T6601. In another aspect of this embodiment, a compressive strength within a range of 150 MPa to 250 MPa is obtained by testing in accordance with JIS T6604. In another aspect of this embodiment, a compressive strength within a range of 150 MPa to 250 MPa is obtained by testing in accordance with JIS T6605.

In another aspect of this embodiment, a compressive strength within a range of 150 MPa to 250 MPa is obtained by testing in accordance with JIS T6608. In another aspect of this embodiment, a compressive strength within a range of 150 MPa to 250 MPa is obtained by testing in accordance with JIS T6609-1. In another aspect of this embodiment, a compressive strength within a range of 150 MPa to 250 MPa is obtained by testing in accordance with JIS T6609-2. In another aspect of this embodiment, a compressive strength within a range of 150 MPa to 250 MPa is obtained by testing in accordance with JIS T6610. In another aspect of this embodiment, a compressive strength within a range of 150 MPa to 250 MPa is obtained by testing in accordance with JIS T6611. In another aspect of this embodiment, a compressive strength within a range of 150 MPa to 250 MPa is obtained by testing in accordance with JIS T6612. In another aspect of this embodiment, a compressive strength within a range of 150 MPa to 250 MPa is obtained by testing in accordance with JIS T6003. In another aspect of this embodiment, a compressive strength within a range of 150 MPa to 250 MPa is obtained by testing in accordance with JIS T6005. In another aspect of this embodiment, a compressive strength within a range of 150 MPa to 250 MPa is obtained by testing in accordance with JIS K 7181.

In this embodiment, the nanofiber content with respect to the total mass of the dental material is preferably within the range of 50% to 80%, more preferably within the range of 55% to 75%, and still more preferably within the range of 60% to 70%. Further, the ratio of biocompatible resin among components other than the nanofiber in the dental material is preferably at least 90% or greater. When an antimicrobial substance and other additives are added, the content thereof can be appropriately determined depending on the type, application, required property or the like of a dental material.

The dental material can thereby be a filler, which has a mechanical strength that is suitable to the site within the organism where the filler is applied, has excellent dimensional stability, and has an excellent antiwear property. Since the compressive strength of the filler according to this embodiment is controllable, excessive load on a tooth when occlusal force is applied is suppressed when the material is applied to the oral cavity. Since the antimicrobial property of the filler according to this embodiment makes it unlikely that a tooth has plaque adhering thereto or is affected by microbes, secondary tooth decay can also be suppressed.

### (7-3. Denture plate material)

In one embodiment, the dental material of the present invention is a dental plate material with a compressive strength within a range of 60 MPa to 100 MPa. In one aspect of this embodiment, a compressive strength within a range of 60 MPa to 100 MPa is obtained by testing in accordance with JIS T6123. In another aspect of this embodiment, a compressive strength within a range of 60 MPa to 100 MPa is obtained by testing in accordance with JIS T6501. In another aspect of this embodiment, a compressive strength within a range of 60 MPa to 100 MPa is obtained by testing in accordance with JIS T6502. In another aspect of this embodiment, a compressive strength within a range of 60 MPa to 100 MPa is obtained by testing in accordance with JIS T6503. In another aspect of this embodiment, a compressive strength within a range of 60 MPa to 100 MPa is obtained by testing in accordance with JIS T6505. In another aspect of this embodiment, a compressive strength within a range of 60 MPa to 100 MPa is obtained by testing in accordance with JIS T6506. In another aspect of this embodiment, a compressive strength within a range of 60 MPa to 100 MPa is obtained by testing in accordance with JIS T6508. In another aspect of this embodiment, a compressive strength within a range of 60 MPa to 100 MPa is obtained by testing in accordance with JIS T6509. In another aspect of this embodiment, a compressive strength within a range of 60 MPa to 100 MPa is obtained by testing in accordance with JIS T6512. In another aspect of this embodiment, a compressive strength within a range of 60 MPa to 100 MPa is obtained by testing in accordance with JIS T6513. In another aspect of this embodiment, a compressive strength within a range of 60 MPa to 100 MPa is obtained by testing in accordance with JIS T6514. In another aspect of this embodiment, a compressive strength within a range of 60 MPa to 100 MPa is obtained by testing in accordance with JIS T6515. In another aspect of this embodiment, a compressive strength within a range of 60 MPa to 100 MPa is obtained by testing in accordance with JIS T6517. In another aspect of this embodiment, a compressive strength within a range of 60 MPa to 100 MPa is obtained by testing in accordance with JIS T6518. In another aspect of this embodiment, a compressive strength within a range of 60 MPa to 100 MPa is obtained by testing in accordance with JIS T6519. In another aspect of this embodiment, a compressive strength within a range of 60 MPa to 100 MPa is obtained by testing in accordance with JIS T6520. In another aspect of this embodiment, a compressive strength within a range of 60 MPa to 100 MPa is obtained by testing in accordance with JIS T6521. In another aspect of this embodiment, a compressive strength within a range of 60 MPa to 100 MPa is obtained by testing in accordance with JIS T6522. In another aspect of this embodiment, a compressive strength within a range of 60 MPa to 100 MPa is obtained by testing in accordance with JIS T6523. In another aspect of this embodiment, a compressive strength within a range of 60 MPa to 100 MPa is obtained by testing in accordance with JIS T6524. In another aspect of this embodiment, a compressive strength within a range of 60 MPa to 100 MPa is obtained by testing in accordance with JIS T6525-1. In another aspect of this embodiment, a compressive strength within a range of 60 MPa to 100 MPa is obtained by testing in accordance with JIS T6525-2. In another aspect of this embodiment, a compressive strength within a range of 60 MPa to 100 MPa is obtained by testing in accordance with JIS T6527. In another aspect of this embodiment, a compressive strength within a range of 60 MPa to 100 MPa is obtained by testing in accordance with JIS T6601. In another aspect of this embodiment, a compressive strength within a range of 60 MPa to 100 MPa is obtained by testing in accordance with JIS T6604. In another aspect of this embodiment, a compressive strength within a range of 60 MPa to 100 MPa is obtained by testing in accordance with JIS T6605. In another aspect of this embodiment, a compressive strength within a range of 60 MPa to 100 MPa is obtained by testing in accordance with JIS T6608. In another aspect of this embodiment, a compressive strength within a range of 60 MPa to 100 MPa is obtained by testing in accordance with JIS T6609-1. In another aspect of this embodiment, a compressive strength within a range of 60 MPa to 100 MPa is obtained by testing in accordance with JIS T6609-2. In another aspect of this embodiment, a compressive strength within a range of 60 MPa to 100 MPa is obtained by testing in accordance with JIS T6610. In another aspect of this embodiment, a compressive strength within a range of 60 MPa to 100 MPa is obtained by testing in accordance with JIS T6611. In another aspect of this embodiment, a compressive strength within a range of 60 MPa to 100 MPa is obtained by testing in accordance with JIS T6612. In another aspect of this embodiment, a compressive strength within a range of 60 MPa to 100 MPa is obtained by testing in accordance with JIS T6003. In another aspect of this embodiment, a compressive strength within a range of 60 MPa to 100 MPa is obtained by testing in accordance with JIS T6005. In another aspect of this embodiment, a compressive strength within a range of 60 MPa to 100 MPa is obtained by testing in accordance with JIS K 7181. More specifically, a compressive strength within a range of 60 MPa to 100 MPa in the denture plate material according to this embodiment is obtained, for example, by testing in accordance with JIS T6501.

In this embodiment, the nanofiber content with respect to the total mass of the dental material is preferably within the range of 30% to 70%, more preferably within the range of 35% to 65%, and still more preferably within the range of 40% to 60%. Further, the ratio of biocompatible resin among components other than the nanofiber in the dental material is preferably at least 90% or greater. When an antimicrobial substance and other additives are added, the content thereof can be appropriately determined depending on the type, application, required property or the like of a dental material.

The dental material can thereby be a dental plate material, which has a mechanical strength that is suitable to the site within the organism where the denture plate material is applied, has excellent dimensional stability, and has an excellent antiwear property. Since a denture plate material and denture material can be made from the same component by applying the denture plate material according to this embodiment, error in the dimension that can arise when the materials are different is suppressed to enable more precise controlling of the dimensions, so that the biocompatibility of dental material as a while is further improved.

### (7-4. Mold repairing material)

In one embodiment, the dental material of the present invention is a mold repairing material with a compressive strength within a range of 30 MPa to 50 MPa. In one aspect of this embodiment, a compressive strength within a range of 30 MPa to 50 MPa is obtained by testing in accordance with JIS T6123. In another aspect of this embodiment, a compressive strength within a range of 30 MPa to 50 MPa is obtained by testing in accordance with JIS T6501. In another aspect of this embodiment, a compressive strength within a range of 30 MPa to 50 MPa is obtained by testing in accordance with JIS T6502.

In another aspect of this embodiment, a compressive strength within a range of 30 MPa to 50 MPa is obtained by testing in accordance with JIS T6503. In another aspect of this embodiment, a compressive strength within a range of 30 MPa to 50 MPa is obtained by testing in accordance with JIS T6505. In another aspect of this embodiment, a compressive strength within a range of 30 MPa to 50 MPa is obtained by testing in accordance with JIS T6506. In another aspect of this embodiment, a compressive strength within a range of 30 MPa to 50 MPa is obtained by testing in accordance with JIS T6508. In another aspect of this embodiment, a compressive strength within a range of 30 MPa to 50 MPa is obtained by testing in accordance with JIS T6509. In another aspect of this embodiment, a compressive strength within a range of 30 MPa to 50 MPa is obtained by testing in accordance with JIS T6512. In another aspect of this embodiment, a compressive strength within a range of 30 MPa to 50 MPa is obtained by testing in accordance with JIS T6513. In another aspect of this embodiment, a compressive strength within a range of 30 MPa to 50 MPa is obtained by testing in accordance with JIS T6514. In another aspect of this embodiment, a compressive strength within a range of 30 MPa to 50 MPa is obtained by testing in accordance with JIS T6515. In another aspect of this embodiment, a compressive strength within a range of 30 MPa to 50 MPa is obtained by testing in accordance with JIS T6517. In another aspect of this embodiment, a compressive strength within a range of 30 MPa to 50 MPa is obtained by testing in accordance with JIS T6518. In another aspect of this embodiment, a compressive strength within a range of 30 MPa to 50 MPa is obtained by testing in accordance with JIS T6519. In another aspect of this embodiment, a compressive strength within a range of 30 MPa to 50 MPa is obtained by testing in accordance with JIS T6520. In another aspect of this embodiment, a compressive strength within a range of 30 MPa to 50 MPa is obtained by testing in accordance with JIS T6521. In another aspect of this embodiment, a compressive strength within a range of 30 MPa to 50 MPa is obtained by testing in accordance with JIS T6522. In another aspect of this embodiment, a compressive strength within a range of 30 MPa to 50 MPa is obtained by testing in accordance with JIS T6523. In another aspect of this embodiment, a compressive strength within a range of 30 MPa to 50 MPa is obtained by testing in accordance with JIS T6524. In another aspect of this embodiment, a compressive strength within a range of 30 MPa to 50 MPa is obtained by testing in accordance with JIS T6525-1. In another aspect of this embodiment, a compressive strength within a range of 30 MPa to 50 MPa is obtained by testing in accordance with JIS T6525-2. In another aspect of this embodiment, a compressive strength within a range of 30 MPa to 50 MPa is obtained by testing in accordance with JIS T6527. In another aspect of this embodiment, a compressive strength within a range of 30 MPa to 50 MPa is obtained by testing in accordance with JIS T6601. In another aspect of this embodiment, a compressive strength within a range of 30 MPa to 50 MPa is obtained by testing in accordance with JIS T6604. In another aspect of this embodiment, a compressive strength within a range of 30 MPa to 50 MPa is obtained by testing in accordance with JIS T6605. In another aspect of this embodiment, a compressive strength within a range of 30 MPa to 50 MPa is obtained by testing in accordance with JIS T6608. In another aspect of this embodiment, a compressive strength within a range of 30 MPa to 50 MPa is obtained by testing in accordance with JIS T6609-1. In another aspect of this embodiment, a compressive strength within a range of 30 MPa to 50 MPa is obtained by testing in accordance with JIS T6609-2. In another aspect of this embodiment, a compressive strength within a range of 30 MPa to 50 MPa is obtained by testing in accordance with JIS T6610. In another aspect of this embodiment, a compressive strength within a range of 30 MPa to 50 MPa is obtained by testing in accordance with JIS T6611. In another aspect of this embodiment, a compressive strength within a range of 30 MPa to 50 MPa is obtained by testing in accordance with JIS T6612. In another aspect of this embodiment, a compressive strength within a range of 30 MPa to 50 MPa is obtained by testing in accordance with JIS T6003. In another aspect of this embodiment, a compressive strength within a range of 30 MPa to 50 MPa is obtained by testing in accordance with JIS T6005. In another aspect of this embodiment, a compressive strength within a range of 30 MPa to 50 MPa is obtained by testing in accordance with JIS K 7181. More specifically, a compressive strength within a range of 30 MPa to 50 MPa in the denture repairing material according to this embodiment is obtained, for example, by testing in accordance with JIS T6514. A compressive strength within a range of 30 MPa to 50 MPa in the mold repairing material according to this embodiment is also obtained by testing in accordance with JIS T6515. A compressive strength within a range of 30 MPa to 50 MPa in the mold repairing material according to this embodiment is also obtained by testing in accordance with JIS T6522. A compressive strength within a range of 30 MPa to 50 MPa in the mold repairing material according to this embodiment is also obtained by testing in accordance with JIS T6524.

In this embodiment, the nanofiber content with respect to the total mass of the dental material is preferably at least 20% or greater, more preferably within the range of 20% to 50%, and still more preferably within the range of 20% to 45%. Further, the ratio of biocompatible resin among components other than the nanofiber in the dental material is preferably at least 90% or greater. When an antimicrobial substance and other additives are added, the content thereof can be appropriately determined depending on the type, application, required property or the like of a dental material.

The dental material can thereby be a mold repairing material, which has a mechanical strength that is suitable to the site within the organism where the mold repairing material is applied, has excellent dimensional stability and has excellent antiwear property. Since the compressive strength of the mold repairing material according to this embodiment is controllable, excessive load on a tooth when occlusal force is applied is suppressed when the material is applied to the oral cavity. Since the antimicrobial property of the mold repairing material according to this embodiment makes it unlikely that a tooth has plaque adhering thereto or is affected by microbes, secondary tooth decay can also be suppressed.

### (7-5. Impression material)

In one embodiment, the dental material of the present invention is an impression material with a compressive strength within a range of 30 MPa to 50 MPa. In one aspect of this embodiment, a compressive strength within a range of 30 MPa to 50 MPa is obtained by testing in accordance with JIS T6123. In another aspect of this embodiment, a compressive strength within a range of 30 MPa to 50 MPa is obtained by testing in accordance with JIS T6501. In another aspect of this embodiment, a compressive strength within a range of 30 MPa to 50 MPa is obtained by testing in accordance with JIS T6502. In another aspect of this embodiment, a compressive strength within a range of 30 MPa to 50 MPa is obtained by testing in accordance with JIS T6503. In another aspect of this embodiment, a compressive strength within a range of 30 MPa to 50 MPa is obtained by testing in accordance with JIS T6505. In another aspect of this embodiment, a compressive strength within a range of 30 MPa to 50 MPa is obtained by testing in accordance with JIS T6506. In another aspect of this embodiment, a compressive strength within a range of 30 MPa to 50 MPa is obtained by testing in accordance with JIS T6508. In another aspect of this embodiment, a compressive strength within a range of 30 MPa to 50 MPa is obtained by testing in accordance with JIS T6509. In another aspect of this embodiment, a compressive strength within a range of 30 MPa to 50 MPa is obtained by testing in accordance with JIS T6512. In another aspect of this embodiment, a compressive strength within a range of 30 MPa to 50 MPa is obtained by testing in accordance with JIS T6513. In another aspect of this embodiment, a compressive strength within a range of 30 MPa to 50 MPa is obtained by testing in accordance with JIS T6514. In another aspect of this embodiment, a compressive strength within a range of 30 MPa to 50 MPa is obtained by testing in accordance with JIS T6515. In another aspect of this embodiment, a compressive strength within a range of 30 MPa to 50 MPa is obtained by testing in accordance with JIS T6517. In another aspect of this embodiment, a compressive strength within a range of 30 MPa to 50 MPa is obtained by testing in accordance with JIS T6518. In another aspect of this embodiment, a compressive strength within a range of 30 MPa to 50 MPa is obtained by testing in accordance with JIS T6519. In another aspect of this embodiment, a compressive strength within a range of 30 MPa to 50 MPa is obtained by testing in accordance with JIS T6520. In another aspect of this embodiment, a compressive strength within a range of 30 MPa to 50 MPa is obtained by testing in accordance with JIS T6521. In another aspect of this embodiment, a compressive strength within a range of 30 MPa to 50 MPa is obtained by testing in accordance with JIS T6522. In another aspect of this embodiment, a compressive strength within a range of 30 MPa to 50 MPa is obtained by testing in accordance with JIS T6523. In another aspect of this embodiment, a compressive strength within a range of 30 MPa to 50 MPa is obtained by testing in accordance with JIS T6524. In another aspect of this embodiment, a compressive strength within a range of 30 MPa to 50 MPa is obtained by testing in accordance with JIS T6525-1. In another aspect of this embodiment, a compressive strength within a range of 30 MPa to 50 MPa is obtained by testing in accordance with JIS T6525-2. In another aspect of this embodiment, a compressive strength within a range of 30 MPa to 50 MPa is obtained by testing in accordance with JIS T6527.

In another aspect of this embodiment, a compressive strength within a range of 30 MPa to 50 MPa is obtained by testing in accordance with JIS T6601. In another aspect of this embodiment, a compressive strength within a range of 30 MPa to 50 MPa is obtained by testing in accordance with JIS T6604. In another aspect of this embodiment, a compressive strength within a range of 30 MPa to 50 MPa is obtained by testing in accordance with JIS T6605. In another aspect of this embodiment, a compressive strength within a range of 30 MPa to 50 MPa is obtained by testing in accordance with JIS T6608. In another aspect of this embodiment, a compressive strength within a range of 30 MPa to 50 MPa is obtained by testing in accordance with JIS T6609-1. In another aspect of this embodiment, a compressive strength within a range of 30 MPa to 50 MPa is obtained by testing in accordance with JIS T6609-2. In another aspect of this embodiment, a compressive strength within a range of 30 MPa to 50 MPa is obtained by testing in accordance with JIS T6610. In another aspect of this embodiment, a compressive strength within a range of 30 MPa to 50 MPa is obtained by testing in accordance with JIS T6611. In another aspect of this embodiment, a compressive strength within a range of 30 MPa to 50 MPa is obtained by testing in accordance with JIS T6612.

In another aspect of this embodiment, a compressive strength within a range of 30 MPa to 50 MPa is obtained by testing in accordance with JIS T6003. In another aspect of this embodiment, a compressive strength within a range of 30 MPa to 50 MPa is obtained by testing in accordance with JIS T6005. In another aspect of this embodiment, a compressive strength within a range of 30 MPa to 50 MPa is obtained by testing in accordance with JIS K 7181. More specifically, a compressive strength within a range of 30 MPa to 50 MPa in the impression material according to this embodiment is obtained, for example, by testing in accordance with JIS T6512. A compressive strength within a range of 30 MPa to 50 MPa in the impression material according to this embodiment is also obtained by testing in accordance with JIS T6513. A compressive strength within a range of 30 MPa to 50 MPa in the impression material according to this embodiment is also obtained by testing in accordance with JIS T6527.

In this embodiment, the nanofiber content with respect to the total mass of the dental material is preferably at least 20% or greater, more preferably within the range of 20% to 50%, and still more preferably within the range of 20% to 45%. Further, the ratio of biocompatible resin among components other than the nanofiber in the dental material is preferably at least 90% or greater. When an antimicrobial substance and other additives are added, the content thereof can be appropriately determined depending on the type, application, required property or the like of a dental material.

The dental material can thereby be an impression material, which has a mechanical strength that is suitable to the site within the organism where the impression material is applied, has excellent dimensional stability, and has an excellent antiwear property. The impression material according to this embodiment has excellent operability while suppressing a change in dimension.

### <Manufacturing method of dental materials>

The dental material of the present invention is made by mixing (melting and kneading) a nanofiber and a biocompatible resin at a predetermined ratio. A commonly known method can be used as the mixing method. For example, a single shaft kneader, multi-shaft kneader or the like can be used. The order of adding a nanofiber and a biocompatible resin, or the timing of melting the same, in the mixing step is not particularly limited. For example, a nanofiber and a biocompatible resin may be melted and kneaded. Alternatively, the biocompatible resin may be melted in advance and then nanofiber may be added and mixed at the time of kneading. When an antimicrobial substance and other additives are added, the order of adding, or the timing of melting the same, can be appropriately adjusted. The mixing temperature is not particularly limited. The temperature can be appropriately determined depending on the type, combination or the like of nanofiber and biocompatible resin. The mixing ratio of a nanofiber and a biocompatible resin can also be adjusted depending on the type, application, required property or the like of a dental material.

A dispersion medium can also be used in the mixing step from the viewpoint of making sure that a nanofiber and a biocompatible resin are dispersed or the like. The dispersion medium can be appropriately selected depending on the type, combination, or the like of a nanofiber and a biocompatible resin. Examples thereof include water, methanol, ethanol, isopropanol, other alcohols, dimethylformamide, N-methyl-2-pyrrolidone (NMP), other amides, mixed solvent thereof, and the like. For example, sodium dodecylbenzenesulfonate, sodium dodecyl sulfate, sodium cholate, sodium deoxycholate, or the like may be added to a dispersion medium as a dispersant in order to disperse a nanofiber and a biocompatible resin more homogeneously while considering the compatibility of the nanofiber and biocompatible resin with the dispersion medium or the like, or another additive may be added depending on the required objective, to the extent that the objective and effect of the present invention are not inhibited.

A dental material obtained in this manner can be used directly or after molding as needed as a dental material of interest. For example, the dental material of the present invention can be molded with high precision in the dimensions by loading (filling) a cavity therewith directly as a filler and curing the material, and then shaping and polishing the details using a grinding instrument or the like. The dental material of the present invention can also be molded into a desired inlay, crown, or the like using a CAD/CAM system. For example, an inlay, crown, or the like of interest can be molded with high precision in the dimensions by setting an external shape of a cavity on a monitor from an optical impression of a cavity after treating a tooth decay captured with a 3D camera and cutting a block of dental material with a disk in a milling chamber.

### <B. Application other than dental material>

The materials comprising a nanofiber and a biocompatible material of the present invention are excellent for not only dental materials but also for other applications. Other than as a dental material, the materials comprising a nanofiber and a biocompatible material of the present invention explained in <A. Dental materials> can also be used for strengthening a cask of a fuel rod, or as a radiation shield, as a reinforcing material for a body of an automobile, a ship, a spacecraft, a space station, a rocket, an aircraft, or a motorcycle, as a space debris post-pulverization adhesive material, as a material for a debris bumper equipped space craft or space station, as a material for a robot frame, as a roofing material comprising a radiation shielding material, or as a construction material.

For the aforementioned applications other than dental materials, a coating agent and/or an additive can be added as needed to the material comprising a nanofiber and a biocompatible material of the present invention explained in <A. Dental material>.

As a nanofiber, the nanofiber of the present invention explained in <A. Dental material> can be used. Examples of nanofibers include, but are not limited to, cellulose nanofibers.

As a biocompatible material, the biocompatible resin of the present invention explained in <A. Dental material> can be used. Examples of biocompatible resin include, but are not limited to, resin selected from the group consisting of methyl methacrylate (MMA), polymethyl methacrylate (PMMA), 2-hydroxyethyl methacrylate (HEMA), tri-n-butylborane (TBB), and 4-methacryloxyethyl trimellitic anhydride (4-META), such as 4-META. In applications other than dental materials, biocompatibility is not important, so that biocompatibility is not always required as long as it is a resin with the same properties as the biocompatible resins explained in <A. Dental material>.

For example, polyuria can be used as a coating agent, but a coating agent is not limited thereto.

For example, lead, tungsten, boron, graphite, graphene, cadmium, and a mixture thereof can be selected as an additive depending on the objective of use. Any other additive may be added to the material of the present invention in accordance with the objective.

The adhesive force of the above material can be enhanced when the material is produced by substituting moisture in a raw material with a monomer of an adhesive material to apply hydrophobic processing, regardless of whether the raw material is a slurry or powder. It is also possible to create a stronger mold by continuously applying vibrations from 20 Hz to 50 Hz, 50 Hz to 120 Hz, and/or 120 Hz to 240 Hz when the material is driven into a mold or frame.

In one embodiment, the above material of the present invention has a strength (typically a compressive strength) that is preferably, but not limited to, 1 GPa to 3 GPa. In the present invention, the strength (typically a compressive strength) is obtained by testing a material of interest in accordance with one or more specifications selected from JIS A1106:2006, JIS A1107:2012, JIS A1108:2006, JIS A1113:2006, JIS A1114:2011, JIS A1132:2014, JIS A1136:1993, JIS A1142:2007, JIS D4610:1993, JIS H7701:2008, JIS R3222:2003, JIS S1200:2012, JIS S1203:1998, JIS S1205:1998, JIS Z8841:1:1993, AST MD953-95, ISO/TS 20746:2016, ISO 75-3:2004, ISO 1752020:2016, ISO1920-4:2005, ISO 1920-5:2004, ISO 2633:1974, ISO 3185:2008, ISO 3186:2008, ISO3193:2008,, ISO-3202:1997, ISO-3203:1993, ISO-7689:2008, ISO-8168:2016, ISO-5856:2008, ISO-5857:2008, ISO-7173:1989, ISO-7176:8:2014, ISO-9152:1998, ISO-9154:2016, ISO-9254:1993, ISO-9255:2008, ISO-9256:1993, ISO-9709:2005, ISO-9845-1:1992, ISO-12258:1998, ISO-12260:2016, ISO-12261:2016, and ISO-1391:2008. For compressive strength, the same testing method may be applied regardless of the type of material, or different testing methods may be applied depending on the type of material.

In one aspect of this embodiment, a strength (typically a compressive strength) within a range of 1 GPa to 3 GPa is obtained by testing in accordance with JIS A1106:2006. In another aspect of this embodiment, a strength (typically a compressive strength) within a range of 1 GPa to 3 GPa is obtained by testing in accordance with JIS A1107:2012. In another aspect of this embodiment, a strength (typically a compressive strength) within a range of 1 GPa to 3 GPa is obtained by testing in accordance with JIS A1108:2006. In another aspect of this embodiment, a strength (typically a compressive strength) within a range of 1 GPa to 3 GPa is obtained by testing in accordance with JIS A1113:2006. In another aspect of this embodiment, a strength (typically a compressive strength) within a range of 1 GPa to 3 GPa is obtained by testing in accordance with JIS A1114:2011. In another aspect of this embodiment, a strength (typically a compressive strength) within a range of 1 GPa to 3 GPa is obtained by testing in accordance with JIS A1132:2014. In another aspect of this embodiment, a strength (typically a compressive strength) within a range of 1 GPa to 3 GPa is obtained by testing in accordance with JIS A1136:1993. In another aspect of this embodiment, a strength (typically a compressive strength) within a range of 1 GPa to 3 GPa is obtained by testing in accordance with JIS A1142:2007. In another aspect of this embodiment, a strength (typically a compressive strength) within a range of 1 GPa to 3 GPa is obtained by testing in accordance with JIS D4610:1993. In another aspect of this embodiment, a strength (typically a compressive strength) within a range of 1 GPa to 3 GPa is obtained by testing in accordance with JIS H7701:2008. In another aspect of this embodiment, a strength (typically a compressive strength) within a range of 1 GPa to 3 GPa is obtained by testing in accordance with JIS R3222:2003. In another aspect of this embodiment, a strength (typically a compressive strength) within a range of 1 GPa to 3 GPa is obtained by testing in accordance with JIS S1200:2012. In another aspect of this embodiment, a strength (typically a compressive strength) within a range of 1 GPa to 3 GPa is obtained by testing in accordance with JIS S1203:1998. In another aspect of this embodiment, a strength (typically a compressive strength) within a range of 1 GPa to 3 GPa is obtained by testing in accordance with JIS S1205:1998. In another aspect of this embodiment, a strength (typically a compressive strength) within a range of 1 GPa to 3 GPa is obtained by testing in accordance with JIS Z8841:1:1993. In another aspect of this embodiment, a strength (typically a compressive strength) within a range of 1 GPa to 3 GPa is obtained by testing in accordance with AST MD953-95. In another aspect of this embodiment, a strength (typically a compressive strength) within a range of 1 GPa to 3 GPa is obtained by testing in accordance with ISO/TS 20746:2016. In another aspect of this embodiment, a strength (typically a compressive strength) within a range of 1 GPa to 3 GPa is obtained by testing in accordance with ISO 75-3:2004. In another aspect of this embodiment, a strength (typically a compressive strength) within a range of 1 GPa to 3 GPa is obtained by testing in accordance with ISO 1752020:2016. In another aspect of this embodiment, a strength (typically a compressive strength) within a range of 1 GPa to 3 GPa is obtained by testing in accordance with ISO1920-4:2005. In another aspect of this embodiment, a strength (typically a compressive strength) within a range of 1 GPa to 3 GPa is obtained by testing in accordance with ISO 1920-5:2004. In another aspect of this embodiment, a strength (typically a compressive strength) within a range of 1 GPa to 3 GPa is obtained by testing in accordance with ISO 2633:1974. In another aspect of this embodiment, a strength (typically a compressive strength) within a range of 1 GPa to 3 GPa is obtained by testing in accordance with ISO 3185:2008. In another aspect of this embodiment, a strength (typically a compressive strength) within a range of 1 GPa to 3 GPa is obtained by testing in accordance with ISO 3186:2008. In another aspect of this embodiment, a strength (typically a compressive strength) within a range of 1 GPa to 3 GPa is obtained by testing in accordance with ISO 3193:2008. In another aspect of this embodiment, a strength (typically a compressive strength) within a range of 1 GPa to 3 GPa is obtained by testing in accordance with ISO-3202:1997. In another aspect of this embodiment, a strength (typically a compressive strength) within a range of 1 GPa to 3 GPa is obtained by testing in accordance with ISO-3203:1993. In another aspect of this embodiment, a strength (typically a compressive strength) within a range of 1 GPa to 3 GPa is obtained by testing in accordance with ISO-7689:2008. In another aspect of this embodiment, a strength (typically a compressive strength) within a range of 1 GPa to 3 GPa is obtained by testing in accordance with ISO-8168:2016. In another aspect of this embodiment, a strength (typically a compressive strength) within a range of 1 GPa to 3 GPa is obtained by testing in accordance with ISO-5856:2008. In another aspect of this embodiment, a strength (typically a compressive strength) within a range of 1 GPa to 3 GPa is obtained by testing in accordance with ISO-5857:2008. In another aspect of this embodiment, a strength (typically a compressive strength) within a range of 1 GPa to 3 GPa is obtained by testing in accordance with ISO-7173:1989. In another aspect of this embodiment, a strength (typically a compressive strength) within a range of 1 GPa to 3 GPa is obtained by testing in accordance with ISO-7176:8:2014. In another aspect of this embodiment, a strength (typically a compressive strength) within a range of 1 GPa to 3 GPa is obtained by testing in accordance with ISO-9152:1998. In another aspect of this embodiment, a strength (typically a compressive strength) within a range of 1 GPa to 3 GPa is obtained by testing in accordance with ISO-9154:2016. In another aspect of this embodiment, a strength (typically a compressive strength) within a range of 1 GPa to 3 GPa is obtained by testing in accordance with ISO-9254:1993. In another aspect of this embodiment, a strength (typically a compressive strength) within a range of 1 GPa to 3 GPa is obtained by testing in accordance with ISO-9255:2008. In another aspect of this embodiment, a strength (typically a compressive strength) within a range of 1 GPa to 3 GPa is obtained by testing in accordance with ISO-9256:1993. In another aspect of this embodiment, a strength (typically a compressive strength) within a range of 1 GPa to 3 GPa is obtained by testing in accordance with ISO-9709:2005. In another aspect of this embodiment, a strength (typically a compressive strength) within a range of 1 GPa to 3 GPa is obtained by testing in accordance with ISO-9845-1:1992. In another aspect of this embodiment, a strength (typically a compressive strength) within a range of 1 GPa to 3 GPa is obtained by testing in accordance with ISO-12258:1998. In another aspect of this embodiment, a strength (typically a compressive strength) within a range of 1 GPa to 3 GPa is obtained by testing in accordance with ISO-12260:2016. In another aspect of this embodiment, a strength (typically a compressive strength) within a range of 1 GPa to 3 GPa is obtained by testing in accordance with ISO-12261:2016. In another aspect of this embodiment, a strength (typically a compressive strength) within a range of 1 GPa to 3 GPa is obtained by testing in accordance with ISO-1391:2008.

While the present invention is further explained in detail with the Examples hereinafter, the present invention is not limited to such Examples.

### [Examples]

### <Example 1>

A dental material is obtained using a cellulose nanofiber as a nanofiber and a methacrylate monomer and polymethacrylate as a biocompatible resin by dispersing and thoroughly stirring the nanofiber and the biocompatible resin in water so that the mass ratio of the nanofiber and the biocompatible resin is 85:15, and then placing the dispersion in a kneader to melt and knead the dispersion for a suitable period of time at a predetermined temperature.

The dental material is molded into a predetermined shape to prepare an implant material, prosthetic material, or denture material.

### <Example 2>

A dental material is obtained in the same manner as Example 1, except for the mass ratio of the nanofiber and biocompatible resin being 83:17.

The dental material is molded into a predetermined shape to prepare an implant material, prosthetic material, or denture material.

### <Example 3>

A dental material is obtained in the same manner as Example 1, except for the mass ratio of the nanofiber and biocompatible resin being 88:12.

The dental material is molded into a predetermined shape to prepare an implant material, prosthetic material, or denture material.

### <Example 4>

A dental material is obtained in the same manner as Example 1, except for the mass ratio of the nanofiber and biocompatible resin being 80:20.

The dental material is molded into a predetermined shape to prepare an implant material, prosthetic material, or denture material.

### <Example 5>

A dental material is obtained in the same manner as Example 1, except for the mass ratio of the nanofiber and biocompatible resin being 75:25.

The dental material is molded into a predetermined shape to prepare an implant material, prosthetic material, or denture material.

### <Example 6>

A dental material is obtained in the same manner as Example 1, except for using a cellulose nanofiber-calcium carbonate composite as the nanofiber.

The dental material is molded into a predetermined shape to prepare an implant material, prosthetic material, or denture material.

### <Example 7>

A dental material is obtained in the same manner as Example 1, except for mixing a cellulose nanofiber and chitosan nanofiber as the nanofiber at a mass ratio of 90:10.

The dental material is molded into a predetermined shape to prepare an implant material, prosthetic material, or denture material.

### <Example 8>

A dental material is obtained in the same manner as Example 1, except for using a microcapsule encapsulating copper ions as an antimicrobial substance, in addition to the nanofiber and biocompatible resin, so that the mass ratio is 85:13:2

The dental material is molded into a predetermined shape to prepare an implant material, prosthetic material, or denture material.

### <Example 9>

A dental material is obtained in the same manner as Example 1, except for the mass ratio of the nanofiber and biocompatible resin being 65:35.

The dental material is prepared as a filler.

### <Example 10>

A dental material is obtained in the same manner as Example 1, except for the mass ratio of the nanofiber and biocompatible resin being 50:50.

The dental material is prepared as a dental plate material.

### <Example 11>

A dental material is obtained in the same manner as Example 1, except for the mass ratio of the nanofiber and biocompatible resin being 30:70.

The dental material is prepared as a mold repairing material.

### <Example 12>

A dental material is obtained in the same manner as Example 1, except for the mass ratio of the nanofiber and biocompatible resin being 20:80.

The dental material is prepared as an impression material.

Each of the dental materials obtained in this manner has excellent resistance to occlusal force in the oral cavity, biologically suitable mechanical strength, excellent dimensional stability, and durability.

The embodiments of the present invention have been disclosed in detail, but the specific embodiments are not limited thereto. Changes in design within the scope of the intent of the present invention and the like are encompassed by the present invention.

## Claims

1. A dental material comprising a nanofiber and a biocompatible resin.

2. The dental material of claim 1, wherein the nanofiber is a cellulose nanofiber.

3. The dental material of claim 2, wherein the nanofiber further comprises one or more of a chitosan nanofiber and a chitin nanofiber.

4. The dental material of any one of claims 1 to 3, wherein the dental material comprises an antimicrobial substance.

5. The dental material of any one of claims 1 to 4, wherein the dental material is an implant material, a prosthetic material, a denture material, a filler, a denture plate material, a mold repairing material, or an impression material.

6. The dental material of claim 5, wherein the dental material is an implant material, a prosthetic material, or a denture material with a compressive strength within a range of 300 MPa to 400 MPa.

7. The dental material of claim 5, wherein the dental material is a filler with a compressive strength within a range of 150 MPa to 250 MPa.

8. The dental material of claim 5, wherein the dental material is a denture plate material with a compressive strength within a range of 60 MPa to 100 MPa.

9. The dental material of claim 5, wherein the dental material is a mold repairing material or an impression material with a compressive strength within a range of 30 MPa to 50 MPa.

10. The dental material of any one of claims 6 to 9, wherein the compressive strength is obtained by testing in accordance with one or more specifications selected from JIS T6123, JIS T6501, JIS T6502, JIS T6503, JIS T6505, JIS T6506, JIS T6508, JIS T6509, JIS T6512, JIS T6513, JIS T6514, JIS T6515, JIS T6517, JIS T6518, JIS T6519, JIS T6520, JIS T6521, JIS T6522, JIS T6523, JIS T6524, JIS T6525-1, JIS T6525-2, JIS T6527, JIS T6601, JIS T6604, JIS T6605, JIS T6608, JIS T6609-1, JIS T6609-2, JIS T6610, JIS T6611, JIS T6612, JIS T6003, JIS T6005, and JIS K 7181.

11. A material comprising a nanofiber and a resin, further comprising a substance selected from the group consisting of:
(1) a coating agent and
(2) an additive selected from the group consisting of lead, tungsten, boron, graphite, graphene, and cadmium.

12. The material of claim 11 for strengthening a cask of a fuel rod, or for use as a radiation shield, as a reinforcing material for a body of an automobile, a ship, a spacecraft, a space station, a rocket, an aircraft, or a motorcycle, as a space debris post-pulverization adhesive material, as a material for a debris bumper equipped space craft or space station, as a material for a robot frame, as a roofing material comprising a radiation shielding material, or as a construction material.

13. The material of claim 11, wherein the nanofiber is a cellulose nanofiber.

14. The material of claim 11, wherein the resin is selected from the group consisting of methyl methacrylate (MMA), polymethyl methacrylate (PMMA), 2-hydroxyethyl methacrylate (HEMA), tri-n-butylborane (TBB), and 4-methacryloxyethyl trimellitic anhydride (4-META).

15. The material of claim 11, wherein the coating agent is polyuria.

16. The material of any one of claims 11 to 15 with a compressive strength within a range of 1 GPa to 3 GPa.

17. The dental material of claim 16, wherein the compressive strength is obtained by testing in accordance with one or more specifications selected from JIS A1106:2006, JIS A1107:2012, JIS A1108:2006, JIS A1113:2006, JIS A1114:2011, JIS A1132:2014, JIS A1136:1993, JIS A1142:2007, JIS D4610:1993, JIS H7701:2008, JIS R3222:2003, JIS S1200:2012, JIS S1203:1998, JIS S1205:1998, JIS Z8841:1:1993, AST MD953-95, ISO/TS 20746:2016, ISO 75-3:2004, ISO 1752020:2016, ISO 1920-4:2005, ISO 1920-5:2004, ISO 2633:1974, ISO 3185:2008, ISO 3186:2008, ISO 3193:2008, ISO-3202:1997. ISO-3203:1993, ISO-7689:2008, ISO-8168:2016, ISO-5856:2008, ISO-5857:2008, ISO-7173,1989, ISO-7176:8:2014, ISO-9152:1998, ISO-9154:2016, ISO-9254:1993, ISO-9255:2008, ISO-9256:1993, ISO-9709:2005, ISO-9845-1:1992, ISO-12258:1998, ISO-12260:2016, ISO-12261:2016, and ISO-1391:2008.
